# EUROPÄISCHE PATENTSCHRIFT

(11) **EP 1 848 338 B1**
(45) Veröffentlichungstag und Bekanntmachung des Hinweises auf die Patenterteilung: **08.09.2010**
(21) Anmeldenummer: 06707907.9
(22) Anmeldetag: 30.01.2006
(51) Int. Cl.: A61B 5/15

(54) **ELEKTROMECHANISCHE STECHHILFE ZUR GEWINNUNG FLÜSSIGER PROBEN**
ELECTROMECHANICAL PRICKING AID FOR TAKING LIQUID SAMPLES
ACCESSOIRE DE PIQURE ELECTROMECANIQUE PERMETTANT D'OBTENIR DES ECHANTILLONS LIQUIDES

(30) Priorität: 03.02.2005 DE 102005005017
(43) Veröffentlichungstag der Anmeldung: 31.10.2007
(73) Patentinhaber: F. Hoffmann-La Roche AG, 4070 Basel (CH); Roche Diagnostics GmbH, 68305 Mannheim (DE)
(72) Erfinder: DECK, Frank, 67150 Niederkirchen (DE)
(74) Vertreter: Stößel, Matthias
(86) Internationale Anmeldenummer: PCT/EP2006/050530
(87) Internationale Veröffentlichungsnummer: WO 2006/082174

(56) Entgegenhaltungen:
- WO-A-2004/060143
- US-A1- 2002 170 823
- US-A1- 2004 098 009
- US-B1- 6 210 420

## Beschreibung

### Gebiet der Erfindung

Die Erfindung betrifft eine portable Stechhilfe zur Gewinnung von flüssigen Proben, insbesondere zur Gewinnung von Blutstropfen zur Bestimmung von Blutglukosekonzentrationen. Weiterhin betrifft die Erfindung ein System zur Gewinnung von flüssigen Proben, insbesondere zur Gewinnung von Blutstropfen zur Bestimmung von Blutglukosekonzentrationen, welches mindestens eine erfindungsgemäße portable Stechhilfe sowie eine Ladestation zur Aufladung mindestens eines Langzeitenergiespeichers der portablen Stechhilfe aufweist.

### Stand der Technik

Die Überwachung der Blutglukosekonzentration ist für Diabetiker ein essentieller Bestandteil des Tagesablaufs. Dabei muss die Blutglukosekonzentration schnell und einfach mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche platzsparend und einfach zu handhaben sind, so dass die Blutglukosekonzentration beispielsweise am Arbeitsplatz oder auch in der Freizeit erfolgen kann.

Die Messung der Blutglukosekonzentration erfordert im Wesentlichen zwei Verfahrensschritte: In einem ersten Schritt wird eine entsprechende flüssige Probe bereitgestellt. Dies erfolgt typischerweise dadurch, dass mittels eines sogenannten Lanzettensystems z. B. mittels einer von einem Federsystem angetriebenen Lanzettennadel ein Hautbereich des Patienten perforiert wird, wodurch ein Blutstropfen generiert wird. Für moderne Messsysteme genügt dabei typischerweise eine Blutmenge von ca. 1,5 µl, teilweise sogar Blutmengen unter 1 µl. Derartige Lanzettensysteme bzw. Stechhilfen sind aus dem Stand der Technik bekannt und sind in verschiedenen Ausführungsformen kommerziell erhältlich. Derartige Lanzettensysteme werden beispielsweise in DE 103 02 501 A1 oder in DE 100 47 419 A1 beschrieben. Auch Stechhilfen mit Magazinsystemen zur Aufnahme und Ausgabe mehrerer Lanzetten sind aus diesen Druckschriften bekannt.

In einem zweiten Schritt wird dann der generierte Blutstropfen auf seine Blutglukosekonzentration hin analysiert. Dabei kommen in der Regel Diagnoseverfahren zum Einsatz, welche beispielsweise optische oder elektrochemische Messverfahren einsetzen. Ein Beispiel eines häufig eingesetzten Messverfahrens nutzt eine spezielle Art elektrochemischer Teststreifen, welche beispielsweise so aufgebaut sein können, dass eine vorgegebene Blutmenge über ein Kapillarsystem zu einem Elektrodensystem geführt wird. Bei diesem Elektrodensystem kann es sich z.B. um Goldelektroden handeln, welche mit einer Beschichtung versehen sind. Die Beschichtung enthält zumeist verschiedene Enzyme und sogenannte Mediatoren und bewirkt, dass sich innerhalb der Probe an den Elektroden Ladungsträger (beispielsweise in Form von Redoxmolekülen) bilden, deren Konzentration abhängig ist von der Blutglukosekonzentration. Die Konzentration dieser Ladungsträger kann mittels der Goldelektroden und einem geeigneten, dem Fachmann bekannten Messsystem beispielsweise mittels einer vergleichsweise einfachen Strom-Spannungs-Messung bestimmt werden, wodurch auf die Blutglukosekonzentration zurückgerechnet werden kann.

Aus der US 2002/0170823 A1 ist ein derartiges Testgerät bekannt, welches für eine Mehrzahl von Stoffanalysen in Körperflüssigkeiten genutzt werden kann, beispielsweise zur Messung von Blutglukosekonzentrationen. Das beschriebene Messgerät weist ein ergonomisches Handgerät auf sowie eine Basisstation, wobei das Handgerät und die Basisstation über eine Schnittstelle Daten austauschen können. Das tragbare Handgerät wird durch eine Reihe von Lithium-Batterien mit Energie versorgt.

Eine portable Stechhilfe gemäß Oberbegriff des Anspruchs 1 ist aus WO-A-2004 060 143 bekannt.

Für den ersten oben beschriebenen Schritt, das Generieren eines Blutstropfens, ist bei Verwendung der aus dem Stand der Technik bekannten Systeme und Stechhilfen in der Regel zunächst ein manuelles Spannen des Lanzettensystems erforderlich. Typischerweise wird bei diesem manuellen Spannen ein Federsystem gespannt. Zu diesem Zweck ist üblicherweise eine Kraftaufwendung des Benutzers erforderlich. Diese Kraftaufwendung ist jedoch mit dem Nachteil verbunden, dass insbesondere Kinder oder motorisch eingeschränkte Menschen derartige Stechhilfen zumeist nicht ohne Hilfe alleine verwenden können oder die Verwendung derartiger Systeme mit erheblichen Unbequemlichkeiten verbunden ist. Weiterhin ist bei manchen dieser Stechhilfe auch aufgrund des erforderlichen Spannvorgangs ein Einhandbetrieb nur bedingt möglich.

### Aufgabe

Es ist daher eine Aufgabe der vorliegenden Erfindung, ein System bereitzustellen, welches die beschriebenen Nachteile des Standes der Technik vermeidet. Insbesondere soll eine portable Stechhilfe zur Verfügung gestellt werden, welche auch für Kinder oder motorisch eingeschränkte Patienten leicht zu bedienen ist.

### Lösung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Es wird eine portable Stechhilfe zur Gewinnung von flüssigen Proben, insbesondere zur Gewinnung von Blutstropfen zur Bestimmung von Blutglukosekonzentrationen, vorgeschlagen. Weiterhin wird ein System zur Gewinnung von flüssigen Proben vorgeschlagen, welches eine erfindungsgemäße portable Stechhilfe aufweist sowie eine Ladestation zur Aufladung mindestens einen Langzeitenergiespeichers der portablen Stechhilfe.

Die erfindungsgemäße portable Stechhilfe weist mindestens ein Lanzettensystem auf, wobei das Lanzettensystem mindestens eine Lanzette und mindestens eine Spannvorrichtung zum Spannen des Lanzettensystems aufweist. Bei diesem Lanzettensystem kann es sich beispielsweise um dem Fachmann aus dem Stand der Technik bekannte Lanzettensysteme handeln, beispielsweise Lanzettensysteme, deren Spannvorrichtung ein Federsystem aufweist (siehe beispielsweise DE 100 47 419 A1 oder DE 103 02 501 A1). Auch bezüglich der Ausgestaltung der Lanzette ist die erfindungsgemäße portable Stechhilfe flexibel, so dass grundsätzlich beliebige, dem Fachmann bekannte Lanzetten eingesetzt werden können. Beispielsweise kann die Lanzette mindestens eine Lanzettennadel aufweisen, insbesondere eine Einweg-Lanzettennadel, welche aus hygienischen Gründen nach einem oder wenigen Einstichvorgängen gegen eine neue Lanzettennadel ausgetauscht wird. Anstelle von Lanzettennadeln kann die Lanzette auch analoge Ausgestaltungen aufweisen, beispielsweise prismenförmige scharfkantige Lanzetten oder Ähnliches. Insbesondere kann die portable Stechhilfe eine einzelne Lanzette oder auch eine Mehrzahl von Lanzetten aufweisen. In letzterem Fall wird vorteilhafter Weise ein Magazin zur Aufnahme und/oder Ausgabe von Lanzetten eingesetzt. Ein derartiges Magazin ist beispielsweise in DE 103 02 501 A1 beschrieben.

Weiterhin weist die portable Stechhilfe mindestens einen elektromechanischen Aktor auf, wobei die Spannvorrichtung durch den mindestens einen elektromechanischen Aktor gespannt werden kann. Dieser mindestens eine elektromechanische Aktor weist vorteilhafter Weise mindestens einen Elektromotor auf, beispielsweise einen Gleichstrommotor. Auch andere elektromechanische Aktoren sind möglich, beispielsweise magnetische Systeme (z.B. Elektromagnete) oder Piezo-Systeme. Der mindestens eine elektromechanische Aktor kann beispielsweise direkt oder über ein oder mehrere Getriebe mit dem mindestens einen Lanzettensystem verbunden sein, insbesondere mit der mindestens einen Spannvorrichtung. Dieses Getriebe kann beispielsweise einen Antrieb über einen oder mehrere Antriebsriemen oder über ein oder mehrere Zahnräder aufweisen.

Weiterhin weist die portable Stechhilfe mindestens einen mit dem mindestens einen elektromechanischen Aktor verbundenen wiederaufladbaren Langzeitenerg-iespeicher zum Speichern elektrischer Energie auf. Unter einem Langzeitenergiespeicher soll dabei insbesondere ein Energiespeicher zum Speichern elektrischer Energie verstanden werden, welcher eine Ladung bzw. elektrische Energie bei fehlender elektrischer Belastung auch noch nach Tagen nahezu unverändert hält. Insbesondere soll die elektrische Energie bzw. Ladung ohne Belastung innerhalb von ca. drei Tagen auf nicht weniger als 40 Prozent der ursprünglichen Energie bzw. Ladung abgefallen sein. Bei diesem Langzeitenergiespeicher kann es sich beispielsweise um eine Batterie handeln. Als besonders vorteilhaft hat es sich dabei erwiesen, wenn Akkumulatoren, insbesondere Lithium-Ionen-Akkumulatoren, und/oder Lithium-Polymer-Akkumulatoren zum Einsatz kommen. Auch Nickel-Cadmium-Akkumulatoren und/oder Nickel-Metallhydrid-Akkumulatoren (NimH) sind einsetzbar. Grundsätzlich sind jedoch auch weitere Typen von Akkumulatoren zusätzlich oder alternativ einsetzbar. So lassen sich beispielsweise auch Kondensatoren mit Langzeit-Speicherwirkung einsetzen, beispielsweise sogenannte "Supercaps" (auch Ultra-Capacitors genannt). Diesen Supercaps ist, ähnlich zu Batterien oder Akkumulatoren, eine gespeicherte elektrische Energie auch teilweise entnehmbar, und die Selbstentladung dieser Bauelemente ist sehr gering. Typische Supercaps besitzen nach 30 Tagen ohne Belastung noch ca. 60-70% ihrer ursprünglichen Ladung. Derartige Bauelemente weisen gegenüber herkömmlichen Akkumulatoren insbesondere auch den Vorteil einer schnellen Aufladbarkeit auf.

Die portable Stechhilfe weist weiterhin mindestens eine von außen zugängliche Schnittstelle auf, wobei der mindestens eine Langzeitenergiespeicher zum Speichern elektrischer Energie mit der mindestens einen Schnittstelle verbunden oder verbindbar ist und über die mindestens eine Schnittstelle mit elektrischer Energie aufladbar ist. Bei dieser mindestens einen Schnittstelle kann es sich beispielsweise um eine oder mehrere Elektroden, beispielsweise Metallelektroden, handeln, welche auf der Außenseite des Gehäuses angeordnet ist bzw. sind. Diese Metallelektroden können dann mit einer entsprechenden Gegenschnittstelle (z. B. einer Ladeschnittstelle bzw. Ladestation, siehe unten) mit elektrischer Energie beaufschlagt werden, z.B. durch Anschluss an eine entsprechende Spannungsquelle. Auf diese Weise lässt sich beispielsweise in regelmäßigen Abständen der mindestens eine Langzeitenergiespeicher der portablen Stechhilfe wieder aufladen.

Alternativ oder zusätzlich kann die mindestens eine Schnittstelle auch eine Vorrichtung zur induktiven Aufladung des mindestens einen Langzeitspeichers aufweisen. Beispielsweise kann die mindestens eine Schnittstelle eine mit dem mindestens einen Langzeitspeicher elektrisch verbundene Sekundärspule eines Transformators und einen Transformatorkern aufweisen, so dass im Wesentlichen durch Aufbringen einer Primärspule auf den Transformatorkern und Anlegen einer Wechselspannung an diese Primärspule der mindestens eine Langzeitspeicher induktiv aufgeladen werden kann. Beispielsweise kann diese Primärspule Bestandteil einer Ladestation sein, in welche die portable Stechhilfe eingesteckt werden kann.

Das Aufladen kann beispielsweise erfolgen, wenn eine Ladezustandsanzeige anzeigt, dass ein Minimalladezustand des mindestens einen Langzeitenergiespeichers unterschritten ist. Als besonders vorteilhaft hat es sich daher erwiesen, wenn die portable Stechhilfe eine Ladezustandsanzeige zum Anzeigen eines elektrischen Ladezustands des mindestens einen Langzeitenergiespeichers zum Speichern elektrischer Energie aufweist. Derartige Ladezustandsanzeigen sind dem Fachmann bekannt und können beispielsweise einfache optische und/oder akustische Anzeigen aufweisen. Insbesondere kann die Ladezustandsanzeige eine optische Segmentanzeige aufweisen, beispielsweise in Form einer oder mehrerer Leuchtdioden, welche den Ladezustand des mindestens einen Langzeitenergiespeichers anzeigt. Weiterhin kann beispielsweise auch eine Warnmeldung an einen Benutzer der portablen Stechhilfe ausgegeben werden, insbesondere eine Warnmeldung in Form eines optischen oder akustischen Signals, wenn der Ladezustand des mindestens einen Langzeitenergiespeichers einen vorgegebenen Minimalladezustand erreicht oder unterschreitet. So kann der Benutzer gewarnt werden, wenn der Ladezustand des mindestens einen Langzeitenergiespeichers nicht mehr ausreicht, um einen Spannvorgang des Lanzettensystems zu bewirken, oder auch bereits, wenn der Ladezustand nur noch für wenige Spannvorgänge (beispielsweise ausreichend für eine tägliche Anzahl an Blutglukosemessungen) ausreicht. Auf diese Weise wird z. B. verhindert, dass der Benutzer bzw. Patient unterwegs (beispielsweise in der Freizeit oder in der Arbeit) aufgrund eines unerwartet erschöpften Langzeitenergiespeichers der portablen Stechhilfe keine Blutglukosemessung durchführen kann.

Die erfindungsgemäße portable Stechhilfe weist gegenüber den aus dem Stand der Technik bekannten Stechhilfen eine Reihe von Vorteilen auf. Insbesondere ist bei der erfindungsgemäßen portablen Stechhilfe kein mechanischer Kraftaufwand durch den Benutzer mehr erforderlich, da das mindestens eine Lanzettensystem der portablen Stechhilfe mittels des mindestens einen elektromechanischen Aktors gespannt wird. Daher ist die erfindungsgemäße portable Stechhilfe auch durch motorisch eingeschränkte Patienten oder durch Kinder komfortabel benutzbar. Auch ein Einhandbetrieb der erfindungsgemäßen portablen Stechhilfe ist problemlos möglich. Unterschreitet der Ladezustand des mindestens einen Langzeitenergiespeichers, insbesondere eines Akkumulators, einen vorgegebenen Minimalwert, so wird der Benutzer bzw. Patient entsprechend gewarnt, so dass der mindestens eine Langzeitenergiespeicher entweder aufgeladen oder ausgetauscht werden kann. Zusätzlich kann optional auch eine Vorrichtung vorgesehen sein, mittels derer die Spannvorrichtung manuell spannbar ist, so dass auch bei entleertem oder nahezu entleertem Langzeitenergiespeicher noch ein Spannen des Lanzettensystems möglich ist, diesmal jedoch unter mechanischem Kraftaufwand des Patienten.

Insbesondere kann die portable Stechhilfe auch so ausgestaltet sein, dass mindestens ein Spannzustandssensor vorgesehen ist, welcher mindestens einen Spannzustand der Spannvorrichtung der portablen Stechhilfe erfasst. Weiterhin können Mittel vorgesehen sein, beispielsweise eine entsprechende elektronische Vorrichtung (z.B. ein Mikrocomputer oder andere elektronische Komponenten), welche den erfassten Spannzustand der Spannvorrichtung analysieren. In Abhängigkeit von dem erfassten Spannzustand des Lanzettensystems kann dann ein Spannvorgang ausgelöst werden. Wird beispielsweise festgestellt, dass sich das Lanzettensystem in einem ungespannten Zustand befindet (z.B. nach einem Auslösen des Lanzettensystems), kann die mindestens eine Spannvorrichtung automatisch wieder gespannt werden. Die portable Stechhilfe ist somit wieder betriebsbereit. Ein weiterer Nutzereingriff ist nicht erforderlich. Diese Weiterbildung der Erfindung ist insbesondere vorteilhaft in Kombination mit der oben beschriebenen Ausgestaltung, bei der die portable Stechhilfe ein Magazin zur Aufnahme einer Mehrzahl von Lanzetten aufweist. Das System kann dann beispielsweise so ausgestaltet sein, dass automatisch bei jedem Spannvorgang des Lanzettensystems eine neue Lanzette ausgewählt wird, wodurch eine unhygienische Mehrfachnutzung derselben Lanzette vermieden werden kann. Alternativ kann die Auswahl der Lanzette auch manuell durch einen Benutzer erfolgen, beispielsweise über einen entsprechenden Drehknopf am Magazin.

Diese Erfindung ist besonders vorteilhaft erweiterbar, indem die erfindungsgemäße portable Stechhilfe in einem System zur Gewinnung von flüssigen Proben, insbesondere zur Gewinnung von Blutstropfen zur Bestimmung von Blutglukosekonzentrationen, eingesetzt wird, welches neben mindestens einer erfindungsgemäßen portablen Stechhilfe weiterhin eine Ladestation zur Aufladung des mindestens einen Langzeitenergiespeichers zum Speichern elektrischer Energie der mindestens einen portablen Stechhilfe aufweist. Dabei können eine oder auch mehrere portable Stechhilfen mit einer Ladestation verbunden werden. Derartige Ladestationen (auch Docking Stations genannt) sind aus verschiedenen Bereichen des Standes der Technik bekannt. Neben der bereits oben beschriebenen Basisstation der US 2002/0170823 A1 sind derartige Systeme beispielsweise auch aus der US 6,524,240 B1 zum Aufladen tragbarer medizinischer Geräte bekannt. Ein Beispiel einer elektronischen Schaltung derartiger Ladestationen, welche verhindert, dass beim Fließen eines Ladestromes ein in die Ladestation eingesetztes portables Gerät in Betrieb gesetzt wird, ist beispielsweise aus der DE 40 36 479 A1 bekannt.

Beispielsweise kann die Ladestation mindestens eine Ladeschnittstelle aufweisen, welche mit mindestens einer elektrischen Energiequelle verbunden ist oder verbindbar ist. Diese Verbindung kann beispielsweise auch über einen oder mehrere Schalter (beispielsweise Wahlschalter oder Ein-/Aus-Schalter) sowie über eine elektronische Schaltung erfolgen, so dass beispielsweise auch eine entsprechende Spannungstransformation, ein Überspannungsschutz und/oder eine entsprechende Zeitschaltung eingesetzt werden kann, wodurch die Handhabung des Systems einfach und sicher ausgestaltet werden kann. Die mindestens eine portable Stechhilfe soll derart mit der Ladestation verbindbar sein, dass die mindestens eine Ladeschnittstelle mit der mindestens einen Schnittstelle der portablen Stechhilfe verbunden ist. Diese Verbindung zwischen der portablen Stechhilfe und der Ladestation kann beispielsweise dadurch erfolgen, dass die portable Stechhilfe in eine entsprechende Aussparung der Ladestation eingesetzt wird, wobei vorteilhafter Weise die mindestens eine portable Stechhilfe beim Einsetzen so ausgerichtet wird, dass die mindestens eine Schnittstelle der portablen Stechhilfe in elektrischem Kontakt mit der mindestens einen Ladeschnittstelle der Ladestation steht.

Im Folgenden wird die Erfindung anhand von Ausführungsbeispielen näher beschrieben. Die Erfindung ist jedoch nicht auf die dargestellten Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:
- Figur 1: eine perspektivische Darstellung einer erfindungsgemäßen portablen Stech- hilfe mit geöffnetem Gehäuse;
- Figur 2: eine perspektivische Darstellung eines erfindungsgemäßen Systems zur Gewinnung von flüssigen Proben mit einer portablen Stechhilfe gemäß Fi- gur 1 und einer Ladestation;
- Figur 3: eine Draufsicht einer Ladestation des Ausführungsbeispiels gemäß Figur 2; und
- Figur 4: einen schematischen Ablaufplan eines Verfahrens zur Gewinnung von Blutstropfen zur Bestimmung von Blutglukosekonzentrationen.

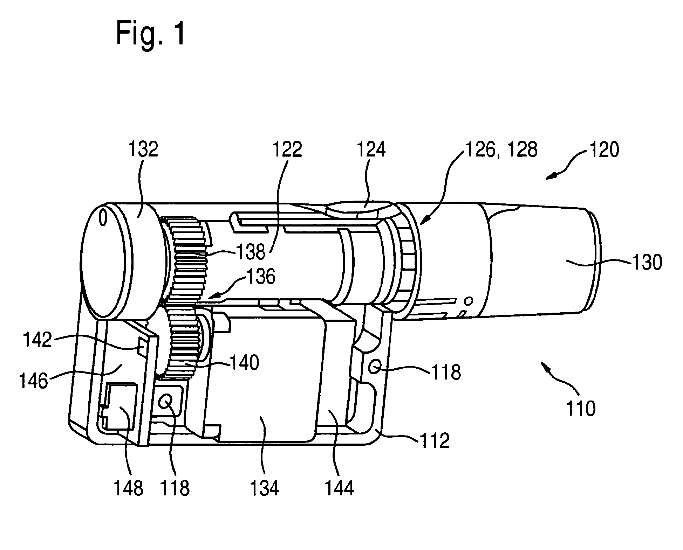

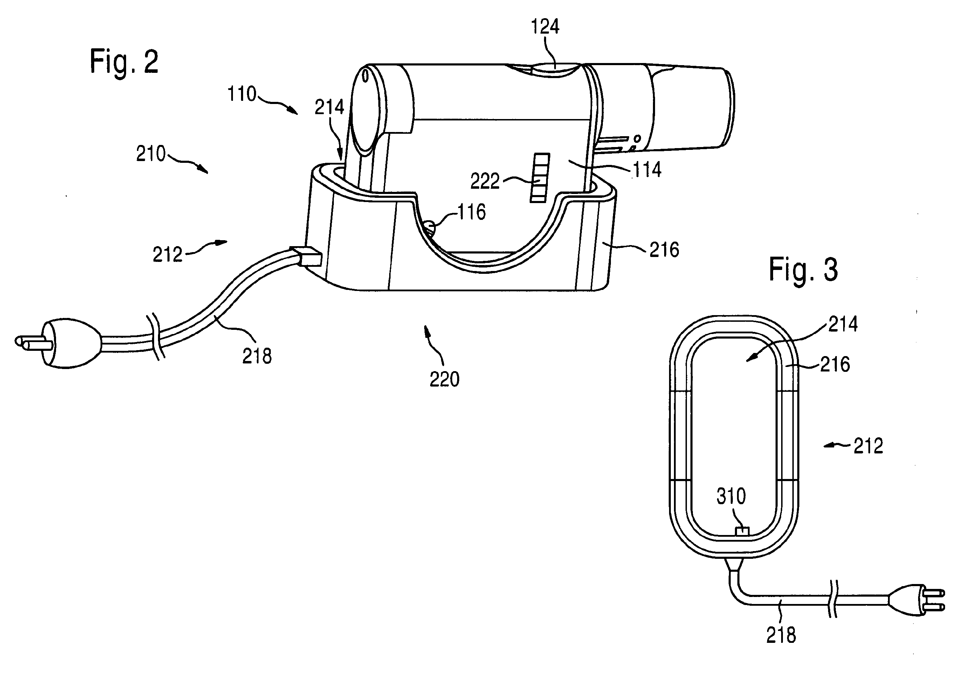

In Figur 1 ist ein Ausführungsbeispiel einer erfindungsgemäßen portablen Stechhilfe 110 in perspektivischer Ansicht dargestellt. Die portable Stechhilfe 110 weist ein Gehäuse 112 auf, welches in der Darstellung gemäß Figur 1 zu Anschauungszwecken geöffnet ist. Zum Öffnen des Gehäuses 112 kann ein Gehäusedeckel 114 (siehe z.B. Figur 2) vom restlichen Gehäuse 112 entfernt werden, indem entsprechende Schrauben 116 (siehe ebenfalls Figur 2) gelöst werden, welche den Gehäusedeckel 114 über entsprechende Gewindebohrungen 118 mit dem restlichen Gehäuse 112 verbinden.

Die portable Stechhilfe 110 weist weiterhin ein Lanzettensystem 120 auf. Die Ausgestaltung und Funktionsweise dieses Lanzettensystems 120 kann beispielsweise analog zu der in DE 102 23 558 A1 offenbarten Ausgestaltung eines Lanzettensystems 120 erfolgen. Auch andere Ausgestaltungen von Lanzettensystemen 120 sind einsetzbar, beispielsweise eine Ausgestaltung gemäß dem in DE 103 12 357 A1 offenbarten Lanzettensystem 120.

Das Lanzettensystem 120 weist eine Spannvorrichtung 122 und einen Auslöseknopf 124 auf Weiterhin weist das Lanzettensystem 120 ein Trommelmagazin 126 (in Figur 1 weitgehend verdeckt) zur Aufnahme mehrerer Einweg-Lanzetten 128 auf. Ein derartiges Trommelmagazin 126 ist in der DE 103 02 501 A1 beschrieben. Weiterhin weist das Lanzettensystem 120 eine drehbar gelagerte Lanzettenkappe 130 auf, in deren (in Figur 1 nicht zu erkennender) Vorderseite ein Austrittsloch für die Lanzette 128 angebracht ist. Die Lanzettenkappe 130 ist abnehmbar ausgestaltet, so dass durch Abnehmen der Lanzettenkappe 130 das Trommelmagazin 126 ausgewechselt werden kann. Durch Verdrehen der Lanzettenkappe 130 kann weiterhin die Einstechtiefe der Lanzetten 128 eingestellt werden. Mittels eines Drehknopfes 132 am der Lanzettenkappe 130 entgegengesetzten Ende des Lanzettensystems 120 kann das Trommelmagazin 126 eingestellt und somit eine neue, unbenutzte Einweg-Lanzette 128 ausgewählt werden. In diesem Ausführungsbeispiel erfolgt die Auswahl einer Einweg-Lanzette 128 manuell durch den Benutzer. Alternativ kann auch eine Vorrichtung vorgesehen sein, mittels derer automatisch nach jedem Einstechvorgang eine neue Einweg-Lanzette 128 aus dem Trommelmagazin 126 ausgewählt wird.

Weiterhin weist die portable Stechhilfe 110 in dem Ausführungsbeispiel gemäß Figur 1 einen Gleichstrommotor 134 auf. Dieser Gleichstrommotor 134 ist über ein in diesem Beispiel zwei Zahnräder 138, 140 aufweisendes Getriebe 136 mit der Spannvorrichtung 122 des Lanzettensystems 120 verbunden. Somit lässt sich mittels des Gleichstrommotors 134 die Spannvorrichtung 122 des Lanzettensystems 120 spannen. Wie oben bereits beschrieben, lassen sich anstelle des Gleichstrommotors 134 auch andere elektromechanische Aktoren einsetzen, beispielsweise magnetische Aktoren, Piezo-Aktoren oder auch komplexere Typen von Motoren, beispielsweise Schrittmotoren.

Weiterhin weist die portable Stechhilfe 110 einen Spannzustandssensor 142 auf, welcher einen Spannzustand der Spannvorrichtung 122 des Lanzettensystems 120 erfassen kann. In diesem Ausführungsbeispiel handelt es sich bei dem Spannzustandssensor 142 um einen Sensor, welcher eine Position des Zahnrads 140 des Getriebes 136 erfasst und über diese Position eine Information darüber gewinnt, ob die Spannvorrichtung 122 gespannt ist oder nicht. Dieser Spannzustandssensor kann beispielsweise auch Bestandteil des Gleichstrommotors 134 sein, wobei beispielsweise eine Winkelstellung des Gleichstrommotors 134 ermittelt wird, wodurch wiederum auf eine Winkelstellung der Zahnräder 138, 140 und/oder des Getriebes 136 zurück geschlossen werden kann. Besonders einfach ist die Erfassung eines Spannungszustands, wenn anstelle eines einfachen Gleichstrommotors 134 ein Schrittmotor eingesetzt wird. Derartige Schrittmotoren sind jedoch vergleichsweise aufwändig. Anstelle eines einzelnen Zustandsensors 142 lassen sich auch mehrere Spannzustandssensoren 142 einsetzen, beispielsweise zwei Spannzustandssensoren 142, wobei beispielsweise ein erster Spannzustandssensor 142 einen gespannten Zustand der Spannvorrichtung 122 detektiert und ein zweiter Spannzustandssensor 142 einen ungespannten Zustand der Spannvorrichtung 122.

Weiterhin weist die portable Stechhilfe 110 einen Lithium-Ionen-Akkumulator 144 sowie eine elektronische Steuerplatine 146 auf. Der Lithium-Ionen-Akkumulator 144 versorgt den Gleichstrommotor 134 und die elektronische Steuerplatine 146 mit elektrischer Energie. Aufgrund der hohen Energiedichte des Lithium-Ionen-Akkumulators 144 sind bei minimaler Akkumulatorgröße typischerweise bis zu ca. einhundert Spannvorgänge der Spannvorrichtung 122 durch den Gleichstrommotor 134 möglich. Weiterhin ist die Selbstentladung derartiger Lithium-Ionen-Akkumulatoren 144 vergleichsweise gering. Insgesamt bewirkt dies, dass bei durchschnittlicher Verwendung der portablen Stechhilfe 110 (typischer Weise zwischen fünf und fünfzehn Mal pro Tag) ein elektrisches Aufladen des Lithium-Ionen-Akkumulators 144 in wöchentlichem Rhythmus oder seltener ausreichend ist.

Die elektronische Steuerplatine 146 der portablen Stechhilfe 110 ist in diesem Ausführungsbeispiel so ausgestaltet, dass der vom Spannzustandssensor 142 erfasste Spannzustand der Spannvorrichtung 122 zum automatischen Spannen des Lanzettensystems 120 genutzt wird. Sobald der Spannzustandssensor 142 detektiert, dass sich die Spannvorrichtung 122 des Lanzettensystems 120 in ungespanntem Zustand befindet (z.B. nach dem Auslösen des Lanzettensystems 120) so wird von der elektronischen Steuerplatine 146 automatisch der Gleichstrommotor 134 entsprechend in Betrieb gesetzt, so dass die Spannvorrichtung 122 wieder gespannt wird und die portable Stechhilfe 110 somit wieder betriebsbereit ist. Auch andere Ausgestaltungen der portablen Stechhilfe 110 sind möglich, beispielsweise der Gestalt, dass ein Spannvorgang der Spannvorrichtung 122 über den Gleichstrommotor 134 erst durch eine entsprechende Aktion eines Benutzers ausgelöst wird, beispielsweise durch Betätigen eines entsprechenden Eingabeknopfes auf einer Oberfläche der portablen Stechhilfe 110.

Weiterhin weist die portable Stechhilfe 110 im Ausführungsbeispiel gemäß Figur 1 eine Schnittstelle 148 auf, welche in diesem Ausführungsbeispiel auf der elektronischen Steuerplatine 146 angeordnet ist und bei geschlossenem Zustand des Gehäuses 112 durch den Gehäusedeckel 114 hindurchragt und somit von außen zugänglich und kontaktierbar ist. Diese Schnittstelle 148 kann insbesondere einen oder mehrere Metallkontakte aufweisen. Über diese Schnittstelle 148 kann der Lithium-Ionen-Akkumulator 144 elektrisch aufgeladen werden. Weiterhin ist auch eine Ausgestaltung der portablen Stechhilfe 110 möglich, bei der Informationen über die Schnittstelle 148 ausgetauscht werden können, beispielsweise um die elektronische Steuerplatine 146 mit Informationen über den Patienten (z.B. Informationen über eine Einstechtiefe des Lanzettensystems 120) zu versorgen.

In Figur 2 ist eine Ausgestaltung eines erfindungsgemäßen Systems 210 zur Gewinnung von flüssigen Proben, insbesondere zur Gewinnung von Blutstropfen zur Bestimmung von Blutglukosekonzentrationen, dargestellt. Das System 210 weist eine portable Stechhilfe 110 gemäß dem in Figur 1 dargestellten Ausführungsbeispiel (in dieser Darstellung mit geschlossenem Gehäusedeckel 114) sowie eine Ladestation 212 auf. In dieser Darstellung ist die portable Stechhilfe 110 in eine entsprechend geformte Aussparung 214 der Ladestation 212 eingesetzt. In Figur 3 ist diese Ladestation 212 noch einmal in Draufsicht dargestellt, wobei die Aussparung 214 deutlich zu erkennen ist. Die Ladestation 212 weist ein Gehäuse 216 auf, in welches die Aussparung 214 eingelassen ist, welche derart ausgestaltet ist, dass das Gehäuse 112 der portablen Stechhilfe 110 derart in die Ladestation 212 eingesetzt werden kann, dass die Schnittstelle 148 der portablen Stechhilfe 110 bei in die Ladestation 212 eingesetzter portabler Stechhilfe 110 in elektrischem Kontakt ist mit einer Ladeschnittstelle 310 der Ladestation 212. Die Ladeschnittstelle 310 steht wiederum in Verbindung mit einem Netzanschluss 218. Diese Verbindung zwischen Ladeschnittstelle 310 und Netzanschluss 218 erfolgt vorteilhafter Weise nicht direkt, sondern über eine entsprechende elektronische Schaltung, welche insbesondere beispielsweise Schalter, einen Überspannungsschutz, einen Spannungstransformator und/oder andere elektronische Bauelemente aufweisen kann. Auf diese Weise ist sichergestellt, dass bei in die Ladestation 212 eingesetzter portabler Stechhilfe 110 der Lithium-Ionen-Akkumulator 144 der portablen Stechhilfe 110 optimal elektrisch aufgeladen und nicht durch falsche Handhabung oder elektrische Störungen (z. B. Netzschwankungen oder Kurzschlüsse) beschädigt wird.

Weiterhin ist die Ladestation 212 mit einer ebenen Unterseite 220 versehen, so dass die Ladestation 212 auch mit eingesetzter portabler Stechhilfe 110 sicher und ohne Kippgefahr auf ebene Oberflächen positioniert werden kann. Auch Ladestationen 212 mit mehreren Aussparungen 214 und Ladeschnittstellen 310 zum gleichzeitigen Aufladen mehrerer portabler Stechhilfen 110 sind möglich, welche beispielsweise im Krankenhausbetrieb eingesetzt werden können.

Weiterhin ist in Figur 2 zu erkennen, dass die portable Stechhilfe 110 in diesem Ausführungsbeispiel auf ihrem Gehäusedeckel 114 eine Ladezustandsanzeige 222 aufweist. Diese Ladezustandsanzeige 222, welche auch an anderen Stellen auf dem Gehäuse 112 positioniert sein kann, ist in diesem Ausführungsbeispiel als Segmentanzeige mit fünf Leuchtdiodensegmenten ausgestaltet, wobei z. B. ein Aufleuchten aller fünf Leuchtdiodensegmente dem höchsten Ladezustand des Lithium-Ionen-Akkumulators 144 entspricht, ein Aufleuchten keines der Segmente der Ladezustandsanzeige 222 einem Ladezustand, in welchem der Lithium-Ionen-Akkumulator 144 vollständig entleert ist. Die Leuchtdiodensegmente können beispielsweise auch verschiedenfarbig ausgestaltet sein, um einen Benutzer auf einen niedrigen Ladezustand hinzuweisen. Auch andere Ausgestaltungen der Ladezustandsanzeige 222 sind möglich (siehe oben). Insbesondere kann die Ladezustandsanzeige 222 von der elektronischen Steuerplatine 146 angesteuert werden.

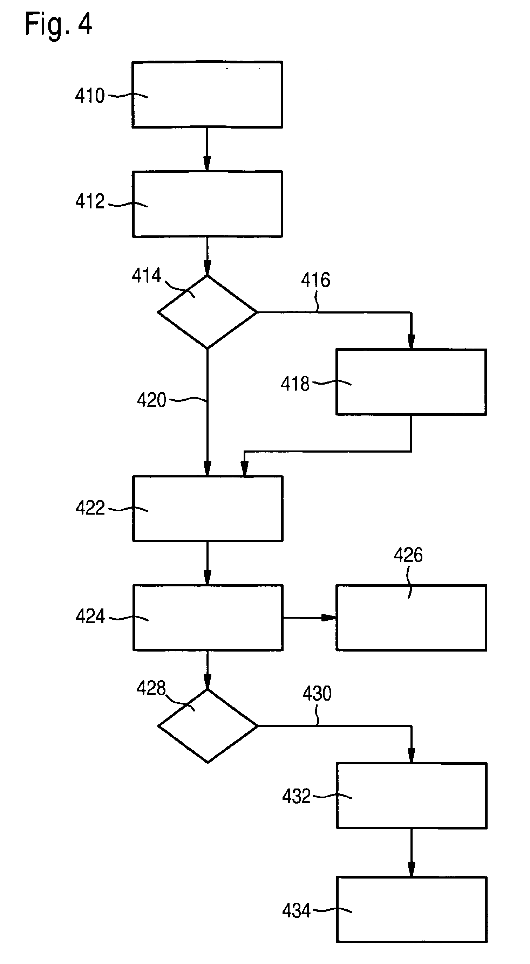

In Figur 4 ist schließlich ein schematischer Ablaufplan einer möglichen Ausgestaltung eines Verfahrens zur Gewinnung von flüssigen Proben, insbesondere zur Gewinnung von Blutstropfen zur Bestimmung von Blutglukosekonzentrationen, dargestellt, wobei insbesondere ein System 210 zur Gewinnung von flüssigen Proben gemäß dem Ausrührungsbeispiel gemäß Figur 2 zum Einsatz kommt. Die in Figur 4 dargestellten Verfahrensschritte müssen nicht notwendiger Weise in der abgebildeten Reihenfolge durchgeführt werden. Auch können weitere, nicht dargestellte Verfahrensschritte durchgeführt werden.

In einem ersten Verfahrensschritt 410 wird eine Einweg-Lanzette 128 ausgewählt, was beispielsweise mittels eines Drehknopfes 132 einer portablen Stechhilfe 110 gemäß dem Ausführungsbeispiel in Figur 1 erfolgen kann. In einem zweiten Verfahrensschritt 412 wird dann mittels eines Spannzustandssensors 142 ein Spannzustand einer Spannvorrichtung 122 eines Lanzettensystems 120 erfasst. Anschließend wird in Verfahrensschritt 414 der erfasste Spannzustand überprüft. Wird festgestellt (Verfahrensschritt 416), dass die Spannvorrichtung 122 sich in einem ungespannten Zustand befindet, so wird Verfahrensschritt 418 durchgeführt, in welchem mittels des Gleichstrommotors 134 die Spannvorrichtung 122 des Lanzettensystems 120 gespannt wird. Wird hingegen in Verfahrensschritt 414 festgestellt, dass sich die Spannvorrichtung 122 bereits in gespanntem Zustand befindet (Verfahrensschritt 420), so wird Verfahrensschritt 418 (Spannen des Lanzettensystems 120) übersprungen. Anschließend ist in beiden Fällen das Lanzettensystem 120 betriebsbereit, und das Lanzettensystem 120 kann (beispielsweise durch Betätigen des Auslöseknopfes 124) ausgelöst werden (Verfahrensschritt 422).

Alternativ oder zusätzlich zu dem in Figur 4 dargestellten Verfahrensablauf kann das Erfassen des Spannungszustands in Verfahrensschritt 412 und die Überprüfung des Spannungszustandes in Verfahrensschritt 414 auch kontinuierlich oder z.B. in regelmäßigen Abständen erfolgen, so dass durch diese kontinuierliche Überprüfung das Lanzettensystem 120 ständig in gespanntem Zustand gehalten wird. Alternativ kann, wie oben bereits beschrieben, das Spannen 418 des Lanzettensystems auch ausgelöst durch eine entsprechende Eingabe eines Benutzers erfolgen.

Anschließend wird in Verfahrensschritt 424 ein Ladezustand des Lithium-Ionen-Akkumulators 144 erfasst. Dieses Erfassen des Ladezustands in Verfahrensschritt 424 muss nicht notwendiger Weise erst nach dem Auslösen 422 des Lanzettensystems 120 erfolgen, sondern die Ladezustandserfassung kann auch beispielsweise kontinuierlich oder in regelmäßigen Abständen oder an anderen Stellen im Verfahrensablauf erfolgen. Der Ladezustand wird in Verfahrensschritt 426, beispielsweise mittels der Ladezustandsanzeige 222, einem Benutzer der portablen Stechhilfe 110 angezeigt. Zusätzlich oder alternativ kann in Verfahrensschritt 428 eine Abfrage durchgeführt werden, in welcher überprüft wird, ob der Ladezustand einen vorgegebenen Minimalladezustand unterschreitet. Ist dies der Fall (Verfahrensschritt 430), so wird in Verfahrensschritt 432 eine Warnmeldung an einen Benutzer ausgegeben, beispielsweise in Form eines akustischen oder optischen Warnsignals. Anschließend erfolgt in Verfahrensschritt 434 eine Aufladung des Lithium-Ionen-Akkumulators 144 mit der portablen Stechhilfe 110, beispielsweise indem die portable Stechhilfe 110 entsprechend der Darstellung gemäß Figur 2 in die Ladestation 212 eingesetzt wird, wobei die Ladestation 212 über den Netzanschluss 218 mit elektrischer Energie versorgt wird.

### Bezugszeichenliste

- 110: portable Stechhilfe
- 112: Gehäuse
- 114: Gehäusedeckel
- 116: Schrauben
- 118: Gewindebohrungen
- 120: Lanzettensystem
- 122: Spannvorrichtung
- 124: Auslöseknopf
- 126: Trommelmagazin
- 128: Einweg-Lanzetten
- 130: Lanzettenkappe
- 132: Drehknopf
- 134: Gleichstrommotor
- 136: Getriebe
- 138: Zahnrad
- 140: Zahnrad
- 142: Spannzustandssensor
- 144: Lithium-Ionen-Akkumulator
- 146: elektronische Steuerplatine
- 148: Schnittstelle

- 210: System zur Gewinnung von flüssigen Proben
- 212: Ladestation
- 214: Aussparung
- 216: Gehäuse der Ladestation
- 218: Netzanschluss
- 220: ebene Unterseite
- 222: Ladezustandsanzeige

- 410: Auswahl einer Lanzette
- 412: Erfassen eines Spannzustandes
- 414: Überprüfung des Spannzustandes
- 416: ungespannter Zustand
- 418: Spannen des Lanzettensystems
- 420: gespannter Zustand
- 422: Auslösen des Lanzettensystems
- 424: Erfassen eines Ladezustands
- 426: Anzeigen des Ladezustands
- 428: Überprüfung, ob Minimalladezustand unterschritten
- 430: Minimalladezustand unterschritten
- 432: Ausgabe Warnmeldung
- 434: Aufladen

## Patentansprüche

1. Portable Stechhilfe (110) zur Gewinnung von flüssigen Proben, insbesondere zur Gewinnung von Blutstropfen zur Bestimmung von Blutglukosekonzentrationen, mit mindestens einem Lanzettensystem (120), wobei das mindestens eine Lanzettensystem (120) mindestens eine Lanzette (128) und mindestens eine Spannvorrichtung (122) zum Spannen des Lanzettensystems (120) aufweist, wobei die portable Stechhilfe (110) folgendes aufweist:
- mindestens einen elektromechanischen Aktor (134), wobei die Spannvorrichtung (122) durch den mindestens einen elektromechanischen Aktor (134) spannbar ist; und
- mindestens einen mit dem mindestens einen elektromechanischen Aktor (134) verbundenen Langzeitenergiespeicher (144) zum Speichern elektrischer Energie, insbesondere einen Lithium-Ionen-Akkumulator (144) und/oder einen Lithium-Polymer-Akkumulator;
**dadurch gekennzeichnet, dass** die portable Stechhilfe (110) weiterhin mindestens eine von außen zugängliche Schnittstelle (148) aufweist, wobei der mindestens eine Langzeitenergiespeicher (144) wiederaufladbar ist und zum Speichern elektrischer Energie mit der mindestens einen Schnittstelle (148) verbunden oder verbindbar ist und über die mindestens eine Schnittstelle (148) mit elektrischer Energie aufladbar ist, insbesondere mittels einer Ladestation (212), wobei die portable Stechhilfe (110) weiterhin mindestens einen Spannzustandssensor (142) zum Erfassen mindestens eines Spannzustandes der Spannvorrichtung (122) und zusätzlich Mittel (146) zum Auslösen eines Spannvorgangs des Lanzettensystems (120) in Abhängigkeit von dem erfassten Spannzustand der Spannvorrichtung (122) aufweist.

2. Portable Stechhilfe (110) gemäß dem vorhergehenden Anspruch, **dadurch gekennzeichnet, dass** der mindestens eine elektromechanische Aktor (134) mindestens einen Elektromotor (134) aufweist.

3. Portable Stechhilfe (110) gemäß dem vorhergehenden Anspruch, **dadurch gekennzeichnet, dass** der mindestens eine Elektromotor (134) mindestens einen Gleichstrommotor (134) aufweist.

4. Portable Stechhilfe (110) gemäß einem der vorhergehenden Ansprüche, **dadurch gekennzeichnet, dass** die Spannvorrichtung (122) ein Federsystem aufweist.

5. Portable Stechhilfe (110) gemäß einem der vorhergehenden Ansprüche mit zusätzlich einem Magazin (126) zur Aufnahme und/oder Ausgabe einer Mehrzahl von Lanzetten (128).

6. Portable Stechhilfe (110) gemäß einem der vorhergehenden Ansprüche mit mindestens einer Ladezustandsanzeige (222) zum Anzeigen eines elektrischen Ladezustands des mindestens einen Langzeitenergiespeichers (144) zum Speichern elektrischer Energie.

7. System (210) zur Gewinnung von flüssigen Proben, insbesondere zur Gewinnung von Blutstropfen zur Bestimmung von Blutglukosekonzentrationen, mit mindestens einer portablen Stechhilfe (110) gemäß einem der vorhergehenden Ansprüche sowie einer Ladestation (212) zur Aufladung des mindestens einen Langzeitenergiespeichers (144) zum Speichern elektrischer Energie der mindestens einen portablen Stechhilfe (110).

8. System (210) zur Gewinnung von flüssigen Proben gemäß dem vorhergehenden Anspruch, **dadurch gekennzeichnet, dass** die Ladestation (212) mindestens eine Ladeschnittstelle (310) aufweist, wobei die mindestens eine Ladeschnittstelle (310) mit mindestens einer elektrischen Energiequelle verbunden oder verbindbar ist und wobei die mindestens eine portable Stechhilfe (110) derart mit der Ladestation (212) verbindbar ist, dass die mindestens eine Ladeschnittstelle (310) mit mindestens einer Schnittstelle (148) der portablen Stechhilfe (110) verbunden ist.

## Claims

1. Portable pricking aid (110) for obtaining liquid samples, more particularly for obtaining drops of blood for determining blood-glucose concentrations, with at least one lancet system (120), wherein the at least one lancet system (120) has at least one lancet (128) and at least one tensioning device (122) for tensioning the lancet system (120), in which the portable pricking aid (110) has the following:
- at least one electromechanical actuator (134), in which the tensioning device (122) can be tensioned by the at least one electromechanical actuator (134); and
- at least one long-term energy storage (144) for storing electrical energy, more particularly a lithium-ion accumulator (144) and/or a lithium-polymer accumulator, connected to the at least one electromechanical actuator (134);
**characterized in that** the portable pricking aid (110) furthermore has at least one interface (148) accessible from the outside, in which the at least one long-term energy storage (144) is rechargeable and is connected or can be connected to the at least one interface (148) for storing electrical energy and can be charged with electrical energy via the at least one interface (148), more particularly by means of a charging station (212), in which the portable pricking aid (110) furthermore has at least one tensioning state sensor (142) for registering at least one tensioning state of the tensioning device (122) and additional means (146) for releasing a tensioning procedure of the lancet system (120) as a function of the registered tensioning state of the tensioning device (122).

2. Portable pricking aid (110) according to the preceding claim, **characterized in that** the at least one electromechanical actuator (134) has at least one electromotor (134).

3. Portable pricking aid (110) according to the preceding claim, **characterized in that** the at least one electromotor (134) has at least one DC motor (134).

4. Portable pricking aid (110) according to one of the preceding claims, **characterized in that** the tensioning device (122) has a spring system.

5. Portable pricking aid (110) according to one of the preceding claims with additionally a magazine (126) for holding and/or dispensing a plurality of lancets (128).

6. Portable pricking aid (110) according to one of the preceding claims with at least one charge-state display (222) for displaying an electrical charge state of the at least one long-term energy storage (144) for storing electrical energy.

7. System (210) for obtaining liquid samples, more particularly for obtaining drops of blood for determining blood-glucose concentrations, with at least one portable pricking aid (110) according to one of the preceding claims and a charging station (212) for charging the at least one long-term energy storage (144) for storing electrical energy of the at least one portable pricking aid (110).

8. System (210) for obtaining liquid samples according to the preceding claim, **characterized in that** the charging station (212) has at least one charging interface (310), in which the at least one charging interface (310) is connected or can be connected to at least one electrical source of energy and in which the at least one portable pricking aid (110) can be connected to the charging station (212) such that the at least one charging interface (310) is connected to at least one interface (148) of the portable pricking aid (110).

## Revendications

1. Accessoire portable de perforation (110) servant à prélever des échantillons liquides, en particulier à prélever des gouttes de sang pour déterminer la concentration du glucose dans le sang, et présentant au moins un système de lancette (120),
le ou les systèmes de lancette (120) présentant au moins une lancette (128) et au moins un dispositif de serrage (122) qui serre le système de lancette (120),
l'auxiliaire portable de perforation (110) présentant :
- au moins un actionneur électromécanique (134), le dispositif de serrage (122) pouvant être serré par le ou les actionneurs électromécaniques (134) et
- au moins un accumulateur durable d'énergie (144) relié à l'actionneur ou aux actionneurs électromécaniques (134) et accumulant de l'énergie électrique, en particulier un accumulateur (144) à ions lithium et/ou un accumulateur à polymère et lithium, **caractérisé en ce que**
l'auxiliaire portable de perforation (110) présente au moins une interface (148) accessible de l'extérieur,
**en ce que** le ou les accumulateurs durables d'énergie (144) sont rechargeables, sont reliés ou peuvent être reliés à la ou aux interfaces (148) pour accumuler de l'énergie électrique et peuvent être rechargés en énergie électrique par l'intermédiaire de la ou des interfaces (148), en particulier au moyen d'un poste de chargement (212),
**en ce que** l'accessoire portable de perforation (110) présente en outre au moins un détecteur (142) d'état de serrage qui détecte au moins un état de serrage du dispositif de serrage (122) et de plus des moyens (146) de déclenchement d'une opération de serrage du système de lancette (120) en fonction de l'état de serrage qui a été détecté sur le dispositif de serrage (122).

2. Accessoire portable de perforation (110) selon la revendication précédente, **caractérisé en ce que** le ou les actionneurs électromécaniques (134) présentent au moins un moteur électrique (134).

3. Accessoire portable de perforation (110) selon la revendication précédente, **caractérisé en ce que** le ou les moteurs électriques (134) présentent au moins un moteur (134) à courant continu.

4. Accessoire portable de perforation (110) selon l'une des revendications précédentes, **caractérisé en ce que** le dispositif de serrage (122) présente un système de ressort.

5. Accessoire portable de perforation (110) selon l'une des revendications précédentes, qui présente de plus un magasin (126) qui loge et/ou délivre plusieurs lancettes (128).

6. Accessoire portable de perforation (110) selon l'une des revendications précédentes, qui présente au moins un affichage (222) de l'état de charge qui affiche l'état de la charge électrique du ou des accumulateurs durables d'énergie (144) servant à accumuler de l'énergie électrique.

7. Système (210) de prélèvement d'échantillons liquides, en particulier de prélèvement de gouttes de sang en vue de déterminer la concentration du glucose dans le sang, et présentant au moins un accessoire portable de perforation (110) selon l'une des revendications précédentes ainsi qu'un poste de chargement (212) servant à recharger la ou les réserves durables d'énergie (144) qui accumulent de l'énergie électrique pour le ou les auxiliaires portables de perforation (110).

8. Système (210) de prélèvement d'échantillons liquides selon la revendication précédente, **caractérisé en ce que** le poste de chargement (212) présente au moins une interface de chargement (310), **en ce que** la ou les interfaces de chargement (310) sont reliées ou peuvent être reliées à la ou aux sources d'énergie et **en ce que** le ou les accessoires portables de perforation (110) peuvent être reliés au poste de chargement (212) en reliant la ou les interfaces de chargement (310) à la ou aux interfaces (148) de l'accessoire portable de perforation (110).
